# EUROPÄISCHE PATENTSCHRIFT

(11) **EP 1 861 118 B1**
(45) Veröffentlichungstag und Bekanntmachung des Hinweises auf die Patenterteilung: **06.05.2015**
(21) Anmeldenummer: 06723678.6
(22) Anmeldetag: 23.03.2006
(51) Int. Cl.: A61K 39/00, A61K 48/00, C07K 14/47

(54) **IDENTIFIZIERUNG VON OBERFLÄCHEN-ASSOZIIERTEN ANTIGENEN FÜR DIE TUMORDIAGNOSE UND -THERAPIE**
IDENTIFICATION OF SURFACE-ASSOCIATED ANTIGENS FOR TUMOR DIAGNOSIS AND THERAPY
IDENTIFICATION D'ANTIGENES DE SURFACE POUR LE DIAGNOSTIC ET LA THERAPIE DE TUMEURS

(30) Priorität: 24.03.2005 DE 102005013846
(43) Veröffentlichungstag der Anmeldung: 05.12.2007
(60) Teilanmeldung: 10010912.3
(73) Patentinhaber: BioNTech AG, 55131 Mainz (DE)
(72) Erfinder: SAHIN, Ugur, 55116 Mainz (DE); TÜRECI, Özlem, 55116 Mainz (DE); KOSLOWSKI, Michael, 65933 Frankfurt (DE); HELFTENBEIN, Gerd, 35329 Gemünden (DE); USENER, Dirk, 65193 Wiesbaden (DE); SCHLÜTER, Volker, 82061 Neuried (DE)
(74) Vertreter: Schnappauf, Georg
(86) Internationale Anmeldenummer: PCT/EP2006/002695
(87) Internationale Veröffentlichungsnummer: WO 2006/100089

(56) Entgegenhaltungen:
- WO-A-98/37094
- WO-A-03/076631
- WO-A-2004/047863
- WO-A-2005/021793
- WO-A2-2005/030250
- DE-A1- 10 344 799
- L. Buonaguro ET AL: "Translating Tumor Antigens into Cancer Vaccines", Clinical and Vaccine Immunology, vol. 18, no. 1, 1 January 2011 (2011-01-01), pages 23-34, XP55009295, ISSN: 1556-6811, DOI: 10.1128/CVI.00286-10

### SEQUENCE LISTING

<110> Ganymed Pharmaceuticals AG
<120> Identifizierung von Oberflächen-assoziierten Antigenen für die Tumordiagnose und -therapie
<130> 342-26PCTEP
<150> DE 10 2005 013 846.2
   <151> 2005-03-24
<160> 8
<170> PatentIn version 3.3
<210> 13
   <211> 2761
   <212> DNA
   <213> Homo sapiens
<400> 13
   ctaggcctca gtctgtctgc atccaggtgc ttattaaaac agtgtgttgc tccacaccgc      60
   ctcgtgttgt ctgttggcgc gctctccggg ttccaaccaa tgcaagagcc ttggggctgg     120
   ccctgaaacc tgcgaggggc ttccgtccac gtccccagtg gacctaccac ccctccatct     180
   gggaaagcag gccacagcag ccggacaaag gaagctcctc agcctctagt cgcctctctg     240
   tgcatgcaca tcggtcactg atctcgccta ctggcacaga cgtgtttatc ggccaaactg     300
   accctcacaa aaagctacca ccgaagtgga caggccccta cactgtgata ctcagcacac     360
   caactgcagt gagagtccga ggactcccca actggatcca tcgcaccagg gtcaagctca     420
   cccccaaggc agcttcttcc tccaaaacat taacagctaa gtgtttgtct gggccaattt     480
   ctcctaccaa gtttaaatta accaacattt ttttcttaaa accaaaacac aaggaagact     540
   aaccacgtgc ttccaggaat ggcctgtatc tacccaacca ctttctatac ctctcttcca     600
   accaaaagtc ttaatatggg aatatccctc accacgatcc taatactgtc agtagctgtc     660
   ctgctgtcca cagcagcccc tccgagctgc cgtgagtgtt atcagtcttt gcactacaga     720
   ggggagatgc aacaatactt tacttaccat actcatatag aaagatcctg ttatggaaac     780
   ttaatcgagg aatgtgttga atcaggaaag agttattata aagtaaagaa tctaggagta     840
   tgtggcagtc gtaatggggc tatttgcccc agagggaagc agtggctttg cttcaccaaa     900
   attggacaat ggggagtaaa cactcaggtg cttgaggaca taaagagaga acagattata     960
   gccaaagcca aagcctcaaa accaacaact ccccctgaaa atcgcccgcg gcatttccat    1020
   tcctttatac aaaaactata agcagatgca tcccttccta agccaggaaa aaatctgttt    1080
   gtagatctag gagaaccatt gtgcttacca tgaatgtgtc caattgttgg gtatgcgggg    1140
   gagctttatg agtgaacagt ggctgtggga cgggatagac attccccctt acttacaggc    1200
   atcccaaaac cccagactca ctttcactcc tcaggaatgc ccgcagtcct ggacacttac    1260
   caacccagta tgagggacgg tgtgcatatc ccgcaagtgg actgataaaa cccatcgcgc    1320
   cgtaggtgaa aacccgtcac caaaccctaa cagtcaatgc ctccatagct gagtggtggc    1380
   caaggttacc ccctggagcc tggtctcctt ctaacttaag ctacctcaat tgtgtcttgt    1440
   caaaaaaggc ctggtactgt acgaacacca ctaaccctta tgccgcatac ctccgcctaa    1500
   gtgtactatg cgacaatcct aggaacacca gctgacaatg gactgccact gacggattcc    1560
   tgtggatatg gggaacccag gcttactcac agctacctta tcactggcaa ggtacttgct    1620
   tcctaggcac aattcaacct ggattctttt tacttccgaa gcaggcgggc aacaccctca    1680
   gagtccctgt gtatgataac cagagaaaaa tgatccttgg aggtaggagg gagccaaaga    1740
   ttgtgagagg acgagtggcc tctgcaacgg atcattgaat actatggtcc tgccacttgg    1800
   gcagaggatg gttcatgggg ttatcgcact cccatatata tgccaaatag agcgattaga    1860
   ctacaagctg ttctagagat aatcactaac caaactgcct cagccctaga aatgctcgcg    1920
   caacaacaaa accaaatgcg cgcggcaatt tatcaaaaca ggctggccct agactactta    1980
   ttagcagaag agggtgcggg ctgtggtaag tttaacatct ccaattgctg tcttaacata    2040
   ggcaataatg gagaagaggt tctggaaatc gcttcaaaca tcagaaaagt agcccgtgta    2100
   ccagtccaaa cctgggaggg atgggaccca gcaaaccttc taggagggtg gttctctaat    2160
   ttaggaggat ttaaaatgct ggtggggaca gtcattttca tcactggggt cctcctgttt    2220
   ctcccctgtg gtatcccatt aaaactcttg ttgaaactac agttaacctc ctgacaatcc    2280
   agatgatgct cctgctacag cggcacgatg gataccaacc cgtctctcaa gaatacccca    2340
   aaaattaagt ttttcttttt ccaaggtgcc cacgccaccc ctatgtcacg cctgaagtag    2400
   ttattgagaa agtcgtccct ttcccctttt ctataaccaa atagacagga atggaagatt    2460
   ctcctcgggg cctgaaagct tgcgggatga ataactcctc ctcctcaggc ccagtcccaa    2520
   ggtacaaact tgcaccagca gcaagatagc agaggcagga agagagctgg ctggaagaca    2580
   cgtaccccct gaagatcaag agggaggtcg ccctggtact acatagcagt cacgttaggc   2640
   tgggacaatt cctgtttaca gaggactata aaacccctgc cccatcctca cttggggctg    2700
   atgccatttt aggcctcagc ctgtctgcat gcaggcgctc attaaaacag catgttgctc    2760
   c                                                                     2761
<210> 14
   <211> 160
   <212> PRT
   <213> Homo sapiens
<400> 14
   Met Ala Cys Ile Tyr Pro Thr Thr Phe Tyr Thr Ser Leu Pro Thr Lys
   1               5                   10                  15
   Ser Leu Asn Met Gly Ile Ser Leu Thr Thr Ile Leu Ile Leu Ser Val
               20                  25                  30
   Ala Val Leu Leu Ser Thr Ala Ala Pro Pro Ser Cys Arg Glu Cys Tyr
           35                  40                  45
   Gln Ser Leu His Tyr Arg Gly Glu Met Gln Gln Tyr Phe Thr Tyr His
       50                  55                  60
   Thr His Ile Glu Arg Ser Cys Tyr Gly Asn Leu Ile Glu Glu Cys Val
   65                  70                  75                  80
   Glu Ser Gly Lys Ser Tyr Tyr Lys Val Lys Asn Leu Gly Val Cys Gly
                   85                  90                  95
   Ser Arg Asn Gly Ala Ile Cys Pro Arg Gly Lys Gln Trp Leu Cys Phe
               100                 105                 110
   Thr Lys Ile Gly Gln Trp Gly Val Asn Thr Gln Val Leu Glu Asp Ile
           115                 120                 125
   Lys Arg Glu Gln Ile Ile Ala Lys Ala Lys Ala Ser Lys Pro Thr Thr
       130                 135                 140
   Pro Pro Glu Asn Arg Pro Arg His Phe His Ser Phe Ile Gln Lys Leu
   145                 150                 155                 160
<210> 15
   <211> 21
   <212> DNA
   <213> künstliche Sequenz
<220>
   <223> Oligonukleotid
<400> 15
   cctctagtcg cctctctgtg c 21
<210> 16
   <211> 17
   <212> DNA
   <213> künstliche Sequenz
<220>
   <223> Oligonukleotid
<400> 16
   accctggtgc gatggat 17
<210> 287
   <211> 19
   <212> PRT
   <213> Homo sapiens
<400> 287
   Met Ala Cys Ile Tyr Pro Thr Thr Phe Tyr Thr Ser Leu Pro Thr Lys
   1               5                   10                  15
   Ser Leu Asn
<210> 288
   <211> 121
   <212> PRT
   <213> Homo sapiens
<400> 288
   Ala Pro Pro Ser Cys Arg Glu Cys Tyr Gln Ser Leu His Tyr Arg Gly
   1               5                   10                  15
   Glu Met Gln Gln Tyr Phe Thr Tyr His Thr His Ile Glu Arg Ser Cys
               20                  25                  30
   Tyr Gly Asn Leu Ile Glu Glu Cys Val Glu Ser Gly Lys Ser Tyr Tyr
           35                  40                  45
   Lys Val Lys Asn Leu Gly Val Cys Gly Ser Arg Asn Gly Ala Ile Cys
       50                  55                  60
   Pro Arg Gly Lys Gln Trp Leu Cys Phe Thr Lys Ile Gly Gln Trp Gly
   65                  70                  75                  80
   Val Asn Thr Gln Val Leu Glu Asp Ile Lys Arg Glu Gln Ile Ile Ala
                   85                  90                  95
   Lys Ala Lys Ala Ser Lys Pro Thr Thr Pro Pro Glu Asn Arg Pro Arg
               100                 105                 110
   His Phe His Ser Phe Ile Gln Lys Leu
           115                 120
<210> 289
   <211> 15
   <212> PRT
   <213> Homo sapiens
<400> 289
   Cys Glu Asn Arg Pro Arg His Phe His Ser Phe Ile Gln Lys Leu
   1 5 10 15
<210> 290
   <211> 13
   <212> PRT
   <213> Homo sapiens
<400> 290
   Cys Ile Tyr Pro Thr Thr Phe Tyr Thr Ser Leu Pro Thr
   1 5 10

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. 1 präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert (Türeci et al., Mol. Med. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Auswertung humaner Protein- und Nukleinsäuredatenbanken im Hinblick auf potenzielle, auf der Zelloberfläche zugängliche, krebsspezifische Antigene. Die Definition der dafür notwendigen Filterkriterien zusammen mit einer Hochdurchsatz-Methodik zur Analyse möglichst aller Proteine bilden den zentralen Bestandteil der Erfindung. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60,1999; Vasmatzis et al., Proc. Natl. Acad. Sci. USA. 95:300-4, 1998; Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Somit liegt der Erfindung eine Strategie zur Identifizierung von differentiell in Tumoren exprimierten Genen zugrunde. Diese kombiniert Datamining von öffentlichen Sequenzbanken (*"in silico"*) mit darauffolgenden evaluierenden laborexperimentellen ("wet bench") Untersuchungen.

Eine kombinierte Strategie basierend auf unterschiedlichen bioinformatischen Skripten ermöglichte erfindungsgemäß die Identifizierung des in Beispiel 1 beschriebenen Gens, das für ein auf der Zelloberfläche zugängliches, krebsspezifisches Antigen kodiert. Die Identifizierung und Bereitstellung dieses Tumor-assoziierten Gens und des dadurch kodierten Genprodukts erfolgte unabhängig von deren immunogener Wirkung.

Wie in Beispiel 1 detailliert dargestellt, kann das erfindungsgemäße Tumor-assoziierte Antigen aufgrund seiner tumorspezifischen Expression als diagnostischer Marker für Krebserkrankungen im Kopf-Hals-Bereich und für Leukämien verwendet werden. Die PCT-Anmeldung WO2005/021793 beschreibt fetale RNA Marker im mütterlichen Blut, die zur pränatalen Diagnose des Down-Syndroms herangezogen werden können. In diesem Zusammenhang wird auch die als "SEQ ID NO: 13" bezeichnete Nukleinsäuresequenz beschrieben, die identisch ist mit der erfindungsgemäßen Nukleinsäuresequenz gemäß SEQ ID NO: 13. Weiterhin beschreibt die PCT-Anmeldung WO98/37094 die Klonierung verschiedenster cDNAs und spekuliert, dass diesen cDNAs und den hiervon abgeleiteten Translationsprodukten eine diagnostische oder therapeutische Bedeutung zukommen könnte. In diesem Zusammenhang wird auch der als "DA306_4" bezeichnete cDNA-Klon erwähnt, der einen offenen Leseraster enthält, der für eine Aminosäuresequenz kodiert, die identisch ist mit der erfindungsgemäßen Aminosäuresequenz gemäß SEQ ID NO: 14. WO98/37094 sind keine Hinweise zu entnehmen, dass ein entsprechendes Protein in tumorspezifischer Weise bei Tumorerkrankungen aus dem Kopf-Hals-Bereich und bei Leukämien exprimiert wird. Schließlich erwähnt zwar die PCT-Anmeldung WO2005/030250, dass das erfindungsgemäße Tumor-assoziierte Antigen in spezifischen Tumorgeweben exprimiert wird. In WO2005/030250 findet sich jedoch kein Hinweis darauf, dass sich Tumorerkrankungen aus dem Kopf-Hals-Bereich und Leukämien durch eine Expression oder Überexpression des erfindungsgemäßen Tumor-assoziierten Antigens auszeichnen.

Das erfindungsgemäß identifizierte Tumor-assoziierte Antigen weist eine Aminosäuresequenz auf, die von einer Nukleinsäure kodiert wird, die eine Nukleinsäuresequenz gemäß SEQ ID NO: 13 des Sequenzprotokolls umfasst. Das erfindungsgemäß identifizierte Tumor-assoziierte Antigen weist eine Aminosäuresequenz gemäß SEQ ID NO: 14 des Sequenzprotokolls auf.

Die vorliegende Erfindung betrifft allgemein die Verwendung des in Beispiel 1 detailliert beschriebenen Tumor-assoziierten Antigens der dafür kodierenden Nukleinsäure oder von Nukleinsäuren, die gegen die kodierenden Nukleinsäuren gerichtet sind, oder von Antikörpern, die gegen das erfindungsgemäß identifizierte Tumor-assoziierte Antigene gerichtet sind, für die Diagnose. Diese Nutzung betrifft z.B. Verfahren zur Diagnose, und Verlaufskontrolle von Karzinomen im Kopf-Hals-Bereich oder von Leukämien.

Die Eigenschaft des erfindungsgemäß identifizierten Tumor-assoziierten Antigens, dass es auf oder an der Zelloberfläche lokalisiert ist, qualifziert es als geeignete Zielestruktur für die Diagnose. Geeignet hierfür ist insbesondere ein Teil des erfindungsgemäß identifizierten Tumor-assoziierten Antigens, der dem Nichttransmembrananteil, insbesondere dem extrazellulären Anteil des Antigens entspricht oder davon umfasst wird. Somit ist erfindungsgemäß ein Teil der erfindungsgemäß identifizierten Tumor-assoziierten Antigene, der dem Nichttransmembrananteil der Antigene entspricht oder davon umfasst ist, oder ein entsprechender Teil der für die erfindungsgemäß identifizierten Antigene kodierenden Nukleinsäuren für eine Diagnose bevorzugt. Ähnlich ist die Verwendung von Antikörpern bevorzugt, die gegen einen Teil der des erfindungsgemäß identifizierten Tumor-assoziierten Antigens gerichtet sind, der dem Nichttransmembrananteil des Antigens entspricht oder davon umfasst ist.

Spleißvarianten sind als Targets für die Diagnostik von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Ausspleißen von einzelnen oder mehreren Exons,
- über Mutation veränderte Spleißregulatorsequenzen (Deletion bzw. Schaffung neuer Donor/Akzeptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker zum Nachweis von Tumorzellen genutzt werden. Die Detektion von Tumorzellen z.B. im Blut, Serum, Knochenmark, Sputum, Bronchial-Lavage, Körpersekreten und Gewebsbiopsien kann z.B. nach Extraktion von Nukleinsäuren durch PCR-Amplifikation mit Spleißvarianten-spezifischen Oligonukleotiden erfolgen.

Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Die Detektion von Tumorzellen kann auch durch Antikörper erfolgen, die tumorspezifisch veränderte Glykosylierungsvarianten erkennen. Für die Generierung von derartigen Antikörpern können Peptidregionen genutzt werden, die sich in Tumorzellen und gesunden Zellen glykosylierungsbedingt unterscheiden. Durch endogene Deglykosylierung von N-gekoppelten Zuckerresten wird Asparagin in Asparaginsäure transformiert. Erfindungsgemäß können daher die hier beschriebenen Proteine tumorspezifisch sequenzverändert sein und damit andere biochemische und Antikörper-Bindungseigenschaften aufweisen. Für die Immunisierung eignen sich besonders die Aminosäuren, die deutliche Epitopunterschiede zu der/den Variante(n) des Genprodukts aufweisen, welche bevorzugt in gesunden Zellen gebildet wird/werden. Der Nachweis der Tumorzellen mit Antikörper kann dabei an einer vom Patienten isolierten Probe oder als Imaging mit intravenös applizierten Antikörpern erfolgen.

Neben der diagnostischen Nutzbarkeit stellen Spleißvarianten, die neue oder veränderte Epitope aufweisen, attraktive Targets für die Immuntherapie dar. Die Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferiert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo- oder Polypeptide, die die Spleißvarianten-spezifischen Epitope beinhalten, oder Nukleinsäuren, die für diese kodieren. Oligo- oder Polypeptide, die die Spleißvarianten-spezifischen Epitope beinhalten, oder Nukleinsäuren, die für diese Polypeptide kodieren, sind auch für die Nutzung als pharmazeutisch wirksame Substanzen bei der aktiven Immuntherapie (Vakzinierung, Vakzintherapie) verwendbar.

Die aberrante Expression von Genen in Tumorzellen kann auf veränderte Methylierungsmuster ihrer Promotoren beruhen (De Smet C. et al., Mol. Cell Biol. 24:4781-90, 2004; De Smet C., et al., Mol. Cell Biol. 19:7327-35, 1999; De Smet C. et al., Proc. Natl. Acad. Sci. USA. 93:7149-53, 1996). Diese Methylierungsunterschiede können als indirekter Marker für den im Tumor veränderten Zustand des entsprechenden Gens genutzt werden. Dementsprechend kann die Zunahme oder Abnahme von Basenmethylierungen im Promotorbereich für diagnostische Zwecke genutzt werden.

Vorliegend werden auch posttranslational modifizierte Proteindomänen wie zum Beispiel Glykosylierungen oder Myristilierungen beschrieben. Diese Art von Modifikationen kann zu einem differentiellen Erkennungsmuster eines Antigens durch z.B. einen Antikörper führen und verschiedene, unter Umständen krankheitsassoziierte Zustände erkennen. Vor allem durch den Einsatz von Antikörpern kann diese Differenzierung eines Antigens sowohl diagnostisch als auch therapeutisch genutzt werden. Für Tumorzellen ist publiziert, dass die tumorassoziierte zelluläre Entartung zu veränderten posttranslationalen Modifikationen führen kann (Durand & Seta, Clin. Chem. 46:795-8052000; Granovsky et al., Nat. Med. 6:306-312, 2000). Insbesondere Glykosylierungsmuster sind auf Tumorzellen stark verändert. Diese speziellen Epitope können diagnostisch Tumorzellen von nicht karzinogenen Zellen unterscheiden. Sollte ein posttranslational modifizierbares Epitop in normalen, nicht entarteten Zellen glykosyliert und in Tumorzellen deglykosyliert sein, erlaubt diese Situation die Entwicklung eines tumorspezifischen therapeutischen Antikörpers.

Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

Des weiteren betrifft die Erfindung Verfahren zur Diagnose oder Überwachung, d.h. Bestimmung der Regression, des Verlaufs und/oder des Ausbruchs, einer Krebserkrankung, die sich durch die Expression oder abnormale Expression eines oder mehrerer Tumor-assoziierter Antigene auszeichnet.

Die erfindungsgemäßen Diagnoseverfahren und/oder Verfahren zur Überwachung betreffen allgemein die Verwendung von Mitteln für den Nachweis und/oder die Bestimmung bzw. Überwachung der Menge (i) einer Nukleinsäure, die für das Tumor-assoziierte Antigen kodiert, oder eines Teils davon und/oder (ii) des Tumor-assoziierten Antigens oder eines Teils davon, in einer aus einem Patienten isolierten biologischen Probe.

In einem Aspekt betrifft die Erfindung ein Verfahren zur Diagnose oder Überwachung einer Krebserkrankung, die sich dadurch auszeichnet, dass ein Tumor-assoziiertes Antigen in Tumorgewebe im Vergleich zu vergleichbarem gesunden Gewebe überexprimiert wird, umfassend
(i) den Nachweis einer Nukleinsäure, die für das Tumor-assoziierte Antigen kodiert, und /oder
(ii) den Nachweis des Tumor-assoziierten Antigens,
in einer aus einem Patienten isolierten biologischen Probe, wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist. Beispielsweise kann das Verfahren (i) die Kontaktierung der biologischen Probe mit einem Mittel umfassen, das spezifisch an die Nukleinsäure, die für das Tumor-assoziierte Antigen kodiert, oder an das Tumor-assoziierte Antigen bindet und (ii) den Nachweis der Komplexbildung zwischen dem Mittel und der Nukleinsäure oder dem Tumor-assoziierten Antigen.

Diagnoseverfahren können das erfindungsgemäß identifizierten Tumor-assoziierten Antigen auch als prognostischen Marker nutzen, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird.

Somit betrifft die Erfindung ein Verfahren zur Bestimmung der Regression, des Verlaufs oder des Ausbruchs einer Krebserkrankung, die sich durch die Expression oder abnormale Expression des erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken.

Ein Nachweis einer Nukleinsäure oder eines Teils davon oder eine Bestimmung bzw. Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. Die Polynukleotid-Sonde umfasst z.B.eine Sequenz von 6-50, insbesondere 10-30, 15-30 oder 20-30 zusammenhängenden Nukleotiden aus der Nukleinsäure.

Im Rahmen des Diagnoseverfahrens erfolgt beispielsweise eine selektive Amplifikation des Promotorbereichs oder eines Teils einer Nukleinsäure, die für das Tumor-assoziierte Antigen kodiert und in Form von genomischer DNA vorliegt, nach Behandlung mit einem bisulfithaltigen Reagenz. Die Nukleinsäure kann vor einer Behandlung mit dem bisulfithaltigen Reagenz aus einer Probe eines zu untersuchenden Patienten isoliert werden. Die bei einer solchen Amplifikation verwendeten Oligonukleotide weisen eine Sequenz auf, die an die mit dem bisulfithaltigen Reagenz behandelte Nukleinsäure binden, vorzugsweise dazu vollständig komplementär sind. Vorzugsweise sind die Oligonukleotide einem unterschiedlichen Grad einer Methylierung der Nukleinsäure angepasst und bedingen differenzierbare Amplifikationsprodukte.

Ein Nachweis eines Tumor-assoziierten Antigens oder eines Teils davon oder eine Bestimmung bzw. Überwachung der Menge eines Tumor-assoziierten Antigens oder eines Teils davon kann mit einem Antikörper erfolgen, der spezifisch an das Tumor-assoziierte Antigen oder den Teil davon bindet.

Das nachzuweisende Tumor-assoziierte Antigen oder ein Teil davon kann in einem Komplex mit einem MHC-Molekül, insbesondere einem HLA-Molekül vorliegen.

Die für einen Nachweis oder für eine Bestimmung bzw. Überwachung verwendete Polynukleotid-Sonde oder, der Antikörper sind beispielsweise nachweisbar markiert. wobei der nachweisbare Marker z.B. ein radioaktiver Marker oder ein Enzymmarker sein kann.

In einem weiteren Aspekt betrifft die Erfindung die Verwendung eines Antikörpers, der an ein Tumor-assoziiertes Antigen bindet und mit einem diagnostischen Mittel gekoppelt ist, zur Herstellung einer pharmazeutischen Zusammensetzung zur Diagnose oder Überwachung einer Krebserkrankung, die sich dadurch auszeichnet, dass das Tumor-assoziierte Antigen in Tumorgewebe im Vergleich zu vergleichbarem gesunden Gewebe überexprimiert wird, wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Der Fachmann kann die Erkenntnisse der erfindungsgemäßen Verfahren auch in Zusammenhang mit Behandlungsverfahren verwenden.

Denkbare Behandlungsverfahren sind Verfahren zur Behandlung eines Patienten mit einer Krebserkrankung, die sich durch die Expression oder abnormale Expression eines Tumor-assoziierten Antigens auszeichnet, umfassend (i) die Identifizierung einer für das erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat) und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Ein solches Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifsch für Zellen des Patienten sind, die das Tumor-assoziierte Antigen oder einen Teil davon exprimieren.

Ein weiterhin denkbares Behandlungsverfahren ist ein Verfahren zur Behandlung einer Krebserkrankung, die sich durch die Expression oder abnormale Expression des erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Oligonukleotide, die mit der erfindungsgemäß identifizierten Nukleinsäure hybridisieren können als genetische Sonden verwendet werden. Nukleinsäuremoleküle in der Form von Oligonukleotid-Primern oder kompetenten Proben, die mit der erfindungsgemäß identifizierten Nukleinsäure hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

Immunogene Fragmente des erfindungsgemäß identifizierten Tumor-assoziierten Antigens können an einen menschlichen HLA-Rezeptor oder menschlichen Antikörper binden. Ein solches Fragment hat z.B. eine Sequenz von mindestens 6, insbesondere mindestens 8, mindestens 10, mindestens 12, mindestens 15, mindestens 20, mindestens 30 oder mindestens 50 Aminosäuren.

In Beispiel 1 werden Peptide beschrieben, die zur Herstellung von Antikörpern verwendet werden können und eine Sequenz aufweisen, ausgewählt aus der Gruppe bestehend aus SEQ ID NOs: 287-290.

In einem weiteren Aspekt betreffen die erfindungsgemäßen Verfahren und Verwendungen ein Mittel, das an das erfindungsgemäß identifizierte Tumor-assoziierte Antigen bindet. In einer bevorzugten Ausführungsform ist das Mittel ein Antikörper. In weiteren Ausführungsformen ist der Antikörper ein chimärer, ein humanisierter Antikörper oder ein Fragment eines Antikörpers. Ein erfindungsgemäßer Antikörper kann ein monoklonaler Antikörper sein. In weiteren Ausführungsformen ist der Antikörper ein chimärer oder humanisierter Antikörper oder ein Fragment eines Antikörpers.

Des weiteren betreffen die erfindungsgemäßen Verwendungen ein Konjugat zwischen einem erfindungsgemäßen Mittel, das an das erfindungsgemäß identifiziertes Tumor-assoziiertes Antigen bindet, oder einem erfindungsgemäßen Antikörper und einem diagnostischen Mittel oder Stoff. Beispielsweise ist das diagnostische Mittel ein Toxin.

In einem weiteren Aspekt betrifft die Erfindung die Verwendung eines Kit zum Nachweis einer Krebserkrankung, die sich dadurch auszeichnet, dass ein Tumor-assoziiertes Antigen in Tumorgewebe im Vergleich zu vergleichbarem gesunden Gewebe überexprimiert wird, wobei der Kit Mittel zum Nachweis
(i) der Nukleinsäure, die für das Tumor-assoziierte Antigen kodiert, und / oder
(ii) des Tumor-assoziierten Antigens,
umfasst, wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist. In einer Ausführungsform sind die Mittel zum Nachweis der Nukleinsäure Nukleinsäuremoleküle für die selektive Amplifikation der Nukleinsäure, die beispielsweise eine Sequenz von 6-50, insbesondere 10-30, 15-30 oder 20-30 zusammenhängenden Nukleotiden aus der Nukleinsäure umfassen.

### Detaillierte Beschreibung der Erfindung

Erfindungsgemäß wird ein Gen ein Gen beschrieben, das in Tumorzellen selektiv exprimiert oder aberrant exprimiert wird und ein Tumor-assoziiertes Antigen darstellt.

Dieses Gene und/oder dessen Genprodukte und/oder dessen Derivate und/oder Teile können Zielstrukturen für therapeutische Ansätze sein. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist beispielsweise Desoxyribonukleinsäure (DNA) oder Ribonukleinsäure (RNA). Nukleinsäuren umfassen beispielsweise genomische DNA, cDNA, mRNA, rekombinant hergestellte und chemisch synthetisierte Moleküle. Eine Nukleinsäure kann als einzelsträngiges oder doppelsträngiges und lineares oder kovalent kreisförmig geschlossenes Molekül vorliegen.

Die beschriebenen Nukleinsäuren können isoliert vorliegen. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0.5 x SSC / 0,1 x SDS bei Temperaturen bis 68°C gewaschen.

Komplementäre Nukleinsäuren weisen beispielsweise mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können alleine oder in Kombination mit anderen Nukleinsäuren, insbesondere heterologen Nukleinsäuren, vorliegen. Eine Nukleinsäure kann funktionell in Verbindung mit Expressionskontrollsequenzen oder regulatorischen Sequenzen vorliegen, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst Promotoren, Enhancer und andere Kontrollelemente, die die Expression eines Gens steuern. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

Prinzipiell können Nukleinsäuren, die für ein erfindungsgemäß identifiziertes Tumor-assoziiertes Antigen kodieren, für eine Transfektion von Wirtszellen eingesetzt werden. Mit Nukleinsäuren ist dabei sowohl rekombinante DNA wie auch RNA gemeint. Rekombinante RNA kann durch in *vitro*-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft jede Zelle, die mit einer exogenen Nukleinsäure transformierbar oder transfizierbar ist. Der Begriff "Wirtszellen" umfasst prokaryontische (z.B. *E*. *coli*) oder eukaryontische (z.B. dendritische Zellen, B-Zellen, CHO-Zellen, COS-Zellen, K562-Zellen, Hefezellen und Insektenzellen). und insbesondere auch Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Beispiele für Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

Erfindungsgemäße Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann ein Duplexmolekül mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. AntisenseOligonukleotide weisen beispielsweise eine Sequenz von 6-50, insbesondere 10-30,15-30 oder 20-30 zusammenhängenden Nukleotiden aus der Ziel-Nukleinsäure auf und können vollständig zu der Ziel-Nukleinsäure oder einem Teil davon komplementär sein. Antisense-Oligonukleotide können beispielsweise mit einer N-terminalen oder 5'-stromaufwärts gelegenen Stelle wie einer Translationsinitiations-, Transkriptionsinitiations- oder Promotorstelle hybridisieren oder mit einer 3'-nicht-translatierten Region oder mRNA-Spleiß-Stelle.

Ein erfindungsgemäßes Oligonukleotid kann aus Ribonukleotiden, Desoxyribonukleotiden oder einer Kombination davon bestehen. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

Alternativ kann ein erfindungsgemäßes Oligonukleotid ein "modifiziertes" Oligonukleotid sein. Dabei kann das Oligonukleotid, um beispielsweise seine Stabilität zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide können isoliert vorliegen. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

"Derivate" von Proteinen oder Polypeptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment des Tumor-assoziierten Antigens kann eine funktionelle Eigenschaft des Polypeptids aufweisen, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Ein Teil oder Fragment eines Tumor-assoziierten Antigens umfasst z.B. eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist beispielsweise ein Teil des Tumor-assoziierten Antigens, der dem Nichttransmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen und/oder für einen Teil oder ein Fragment des Tumor-assoziierten Antigens wie vorstehend definiert kodiert. Vorzugsweise ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die im Rahmen der erfindungsgemäßen Verfahren und Verwendungen eingesetzten Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die im Rahmen von diagnostischen Anwendungen eingesetzt werden. Dazu kann sowohl mit dem gesamten Protein, mit extrazellulären Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of modern Immunology, Wiley & Sons, Inc., New York; Roitt, 1. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2-und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1 - und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Vorzugsweise ist ein erfindungsgemäß verwendeter Antikörper gegen eine Sequenz gemäß SEQ ID NOs 14.

Vorliegend werden auch Bindestoffe wie z.B. Polypeptide beschrieben, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht peptidischen synthetischen Resten hergestellt werden. Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "two-hybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluor und Gadolinium.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, wober der Begriff "Krebs" erfindungsgemäß Leukämien, Seminome, Melanome, Teratome, Gliome, Darm-, Colon-, Rektal-, Kolorektal-, Magen-, Gastrointestinal-, Speiseröhren-, Hals, Nasen, Ohren (HNO)-, Nieren-, Nebennieren-, Schilddrüsen-, Lymphknoten-, Brust-, Prostata-, Gebärmutter-, Ovarial-, Endometrial-, Leber, Pankreas-, Haut-, Gehirn- und Lungenkrebs und deren Metastasen umfasst.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-. Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben.

### Abbildungen:

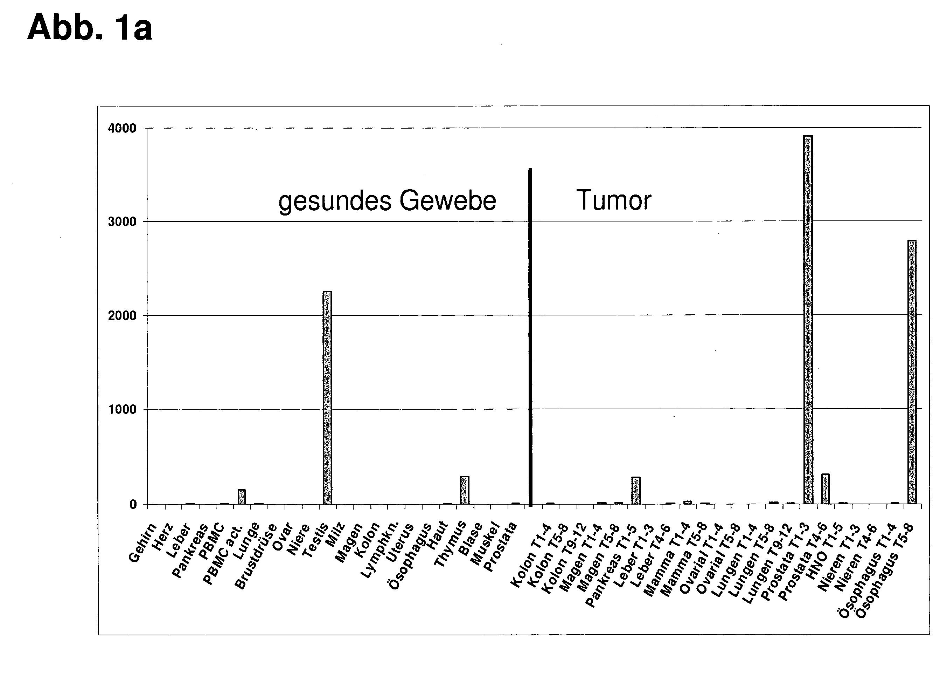

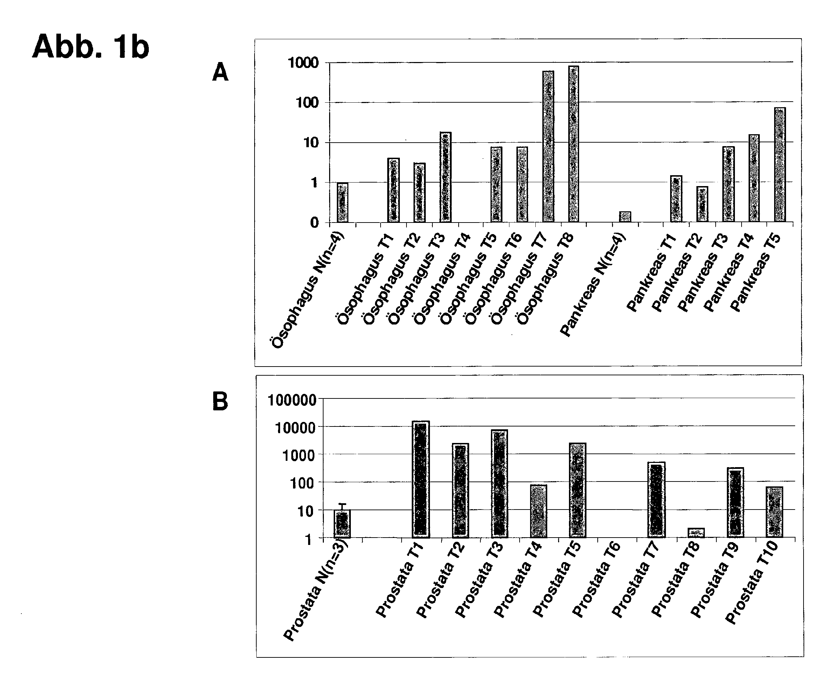

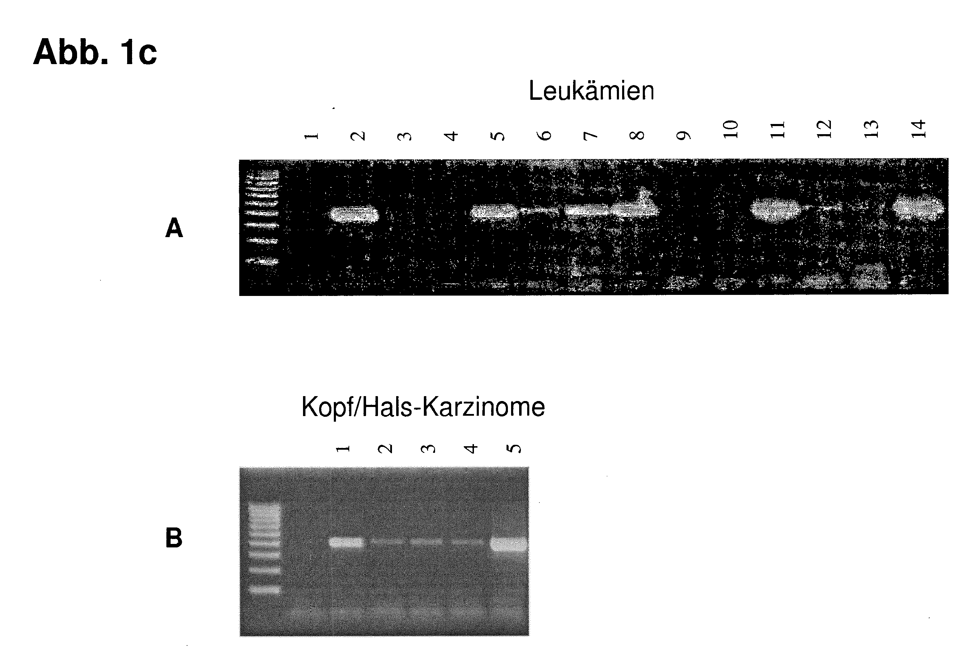

**Abb. 1a****, b: qPCR-Analyse der LOC90625-spezifischen Expression in Normal- und Tumorgeweben.**
**1a:** Quantitative Expressionsanalyse von LOC90625 in Normalgeweben (links) und Tumorgeweben (Pools aus jeweils 3-5 Einzelproben, rechts). Lineare Darstellung der relativen Expression (-fache Aktivierung).
**1b:** Logarithmische Darstellung der Expression in Normal- und Tumorgeweben.
**1c:** RT-PCR-Expressionsanalyse von LOC90625 in A: Leukämien (n=14) und **B**: Kopf/Hals-Karzinomen (n=5).

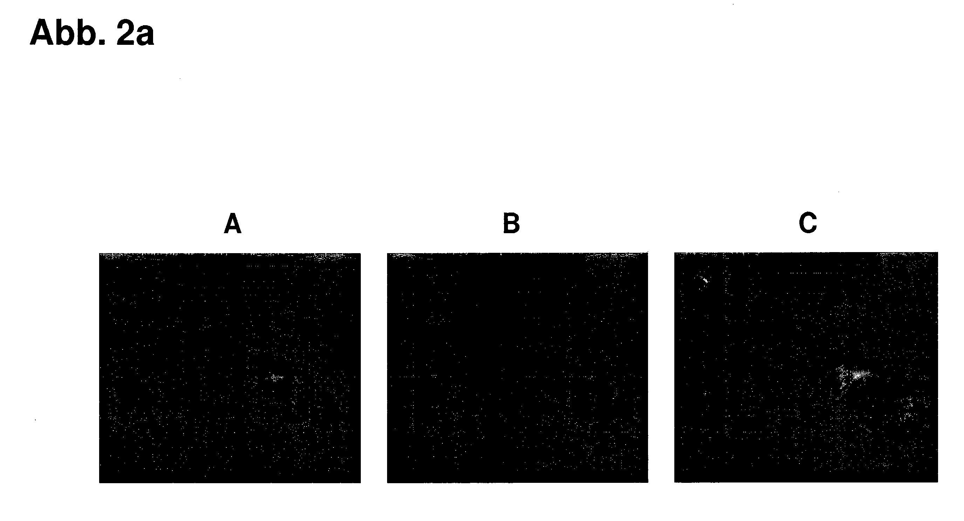

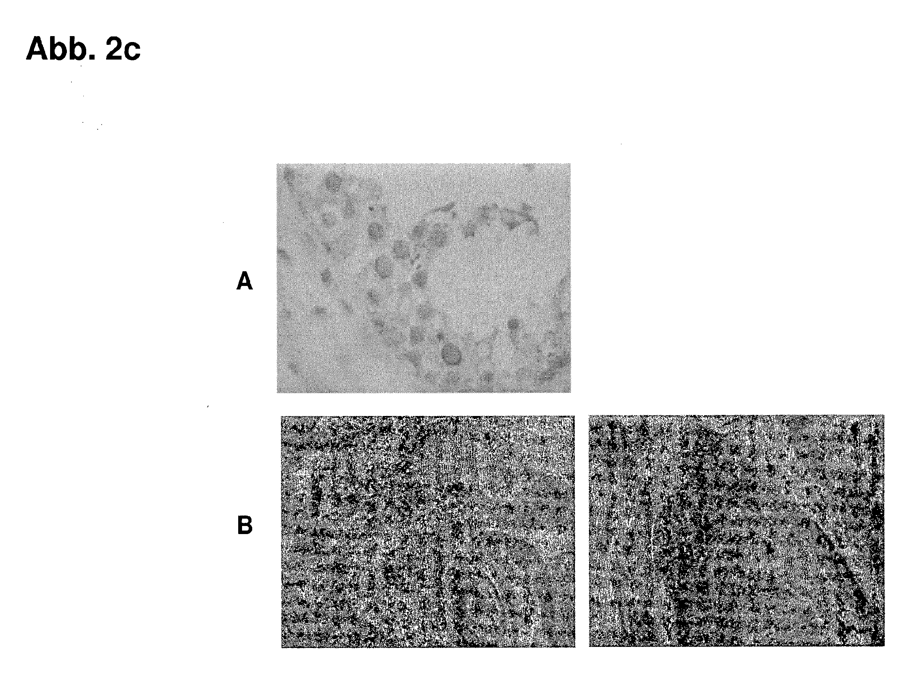

**Abb. 2a****: Färbung von LOC90625-Transfektanten mit spezifischen Antikörpern.** Zellen wurden mit dem spezifischen Gen als Fusionskonstrukt mit eGFP transfiziert **(A)** und mit einem spezifischen Antikörper gegen dieses Genprodukt angefärbt **(B).** Spezifität wird durch die Überlagerung (C) der Färbungen mittels Immunfluoreszenz nachgewiesen.

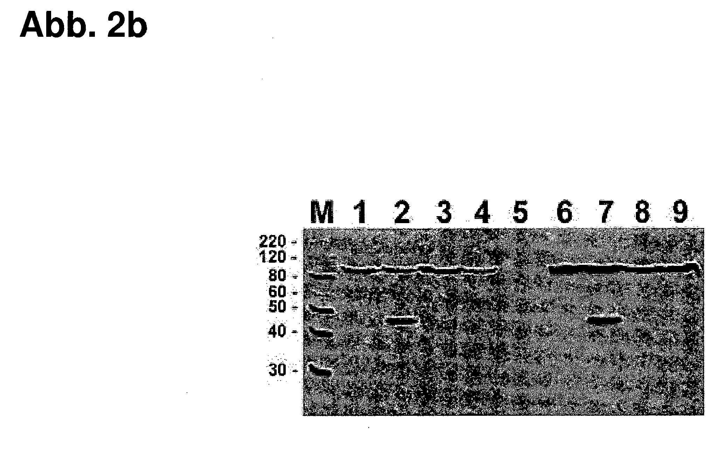

**Abb. 2b****: Färbung von LOC90625-Transfektanten mit spezifischen Antikörpern im Western Blot.** Spuren 1 & 6: 293-Zellen, Spuren 2 & 7: 293-Zellen, transfiziert mit LOC90625 und getaggt mit GFP, Spuren 3, 4, 9 & 8: 293-Zellen, transfiziert mit LOC90625 allein.
**Abb. 2c****: Immunhistochemie in Gewebeschnitten mit LOC90625-Antikörpern.** Keimzellen des Hodens sind die einzigen normalen Zellen, die dieses Genprodukt exprimieren. Tumoren wie das hier dargestellte Prostatakarzinom exprimieren ebenfalls.

### Beispiele:

### Material und Methoden

Die Begriffe *"in silico* ", und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### A. Datamining-basierte Strategie zur Identifizierung von Tumor-assoziierten Antigenen

Erfindungsgemäß wurden öffentliche humane Protein- und Nukleinsäuredatenbanken im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildeten den zentralen Bestandteil dieser Strategie.

Den Ausgangspunkt bildeten die hauptsächlich aus dem humanen Genomprojekt vorhergesagten potenziellen Gene, die in der "RefSeq" Datenbank (Pruitt et al., Trends Genet. Jan;16(1):44-47, 2000) des "National Center for Biotechnology Information" (NCBI) als rein modellhafte Protein- (XP-) bzw. mRNA-Einträge (XM-) abgelegt sind. In einem weiteren Ansatz wurden auch die validierten Proteineinträge (NP-) bzw. die korrespondierenden mRNAs (NM-) derselben Datenbank in gleicher Weise analysiert. Dem Grundprinzip (hypothetisches) Gen zu mRNA zu Protein folgend wurden die Proteine unter Kombination mehrerer Prädiktionsprogramme für Proteinanalyse zunächst auf das Vorhanden sein von Transmembrandomänen hin untersucht. Aus der humanen XP-Fraktion der "RefSeq" Datenbank wurden insgesamt 19.544 Einträge analysiert, wobei 2.025 hypothetische Proteine den Filterkriterien genügten. Die humane NP-Fraktion lieferte insgesamt 19.110 Einträge mit einem Anteil von 4.634 gefilterten Proteinen.

Die korrespondierende mRNA jedes dieser 2.025 bzw. 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche stringent eingestellt. Insgesamt 1.270 hypothetische mRNAs erzielten dabei mindestens einen Treffer in der EST-Datenbank, wobei die Anzahl der Treffer in Einzelfällen mehr als 1.000 betrug.

Für jeden Einzelnen dieser validen Treffer wurde anschließend die gewebsspezifische Herkunft der zugrunde liegenden cDNA Bibliothek sowie der Name der Bibliothek ermittelt. Die daraus resultierenden Gewebe wurden in vier verschiedene Gruppen eingeteilt, die von dispensiblen Organen (Gruppe 3) bis hin zu absolut lebensnotwendigen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST Treffer ausschließlich Krebsgewebe entstammten, gefolgt von denjenigen, die darüber hinaus noch in Geweben dispensibler Organe der Gruppe 3 zu finden sind. Ein weiteres Kriterium für die Aussagekraft dieser Verteilung bildete die Anzahl der unabhängigen cDNA-Bibliotheken, aus denen die ESTs gewonnen wurden und die in einer eigenen Spalte in der Tabelle festgehalten wurde.

Da es sich bei den im ersten Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit Hilfe des Programms "Spidey" (Wheelan et al., Genome Res. 11(11):1952-1957, 2001) dem vorhergesagten Genlokus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu den erfindungsgemäßen Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### B. Validierungsstrategie der durch in silico Analyse identifizierten Tumor-assoziierten Antigene

Zur Nutzung der Targets für immuntherapeutische Zwecke (Antikörpertherapie mittels monoklonaler Antikörper, Vakzinierung, T-Zell Rezeptor-vermittelte therapeutische Ansätze; vgl. EP-B-0 879 282) oder andere zielgerichtete Ansätze (small compounds, siRNA etc.) bei der Krebstherapie sowie für diagnostische Fragestellungen ist die Validierung der erfindungsgemäß identifizierten Targets von zentraler Bedeutung. Die Validierung erfolgt dabei durch Expressionsanalyse sowohl auf RNA als auch auf Proteinebene.

### 1. Untersuchung der RNA Expression

Die erste Charakterisierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittels Guanidiniumisothiocyanat wird die RNA mit saurem Phenol extrahiert, anschließend die RNA mit Isopropanol gefällt und in DEPC-behandeltes Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA Synthese erfolgt dabei mit Hilfe von zufälligen Hexamcren (z.B. Roche Diagnostics) nach Standardprotokollen des jeweiligen Herstellers. Zur Qualitätskontrolle werden die cDNAs mit Primern in 30 Zyklen amplifiziert, die spezifisch für das nur gering exprimierte p53 Gen sind. Nur p53 positive cDNA Proben werden für die weiteren Reaktionsschritte verwendet.

Zur detaillierten Analyse der Targets wird auf Basis eines cDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polymerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionsbuffer (Endkonzentration 1 x, abhängig vom Hersteller der DNA-Polymerase) und je 0,3 mM des gen-spezifischen "sense"- und "antisense"-Primers.

Die spezifischen Primer des Zielgens werden, soweit möglich, so ausgewählt, das sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzyms zu aktivieren. Anschließend wird die DNA in 35 Zyklen amplifiziert (1 min 95°C, 1 min Primer spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative real time PCR erfolgen. Zu dieser Analyse sind inzwischen verschiedene Analysesysteme erhältlich, die bekanntesten sind das ABI PRISM Sequence detection system (TaqMan, Applied Biosystems), der iCycler (Biorad) sowie der Light cycler (Roche Diagnostics). Wie oben beschrieben wird ein spezifischer PCR Ansatz einem Lauf in den "real time"-PCR-Geräten unterzogen. Durch Zusatz eines DNA interkalierenden Farbstoffes (z.B Ethidiumbromid, CybrGreen) wird die neu synthetisierte DNA durch spezifische Lichtanregung (nach Angaben der Farbstoffhersteller) sichtbar gemacht. Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR Ansatzes erfolgt durch Messung eines "housekeeping Gens" (z.B. 18S RNA, ß-Actin). Alternative Strategien über Fluoreszenz- markierte DNA-Sonden erlauben ebenfalls die quantitative Bestimmung des Zielgens aus einer spezifischen Gewebeprobe (siehe TaqMan Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Prädiktion des Proteins

Viele erfindungsgemäß gefundene Gene (insbesondere aus der XM Domäne der RefSeq) sind Gen-Neuentdeckungen, für die das Volllänge-Gen kloniert, das offene Leseraster ermittelt und die Proteinsequenz abgeleitet und analysiert werden muss.

Für die Volllängeklonierung der Sequenz haben wir gängige Protokolle zur "Rapid amplification of cDNA ends", sowie Screening von cDNA Expressionsbanken mit gen-spezifischen Sonden verwendet *(*Sambrook et al., Molekular Cloning: A Laboratory Manual, 2. Auflage (1989), Cold Spring Harbor Laboratory Press, Cold Spring Harbor, N.Y.). Nach Zusammensetzung der so gefundenen Fragmente wurden potentielle Offene Leseraster (ORF) durch gängige Prädiktionsprogramme prädiziert. Da durch die Position des PolyA-Schwanzes und Polyadenylierungs-Motiven die Orientierung des potentiellen Genproduktes vorgegeben wird, verbleiben von möglichen 6 Leserastern nur noch die 3 der jeweiligen Orientierung. Oft ergibt sich aus diesen nur ein hinreichend großes offenes Leseraster, das für ein Protein kodieren kann, während die anderen Leseraster zu viele Stop-Codons aufweisen und für kein realistisches Proteine kodieren würden. Bei alternativen offenen Leserastem unterstützt die Berücksichtigung der Kozak-Kriterien für optimale Transkriptions-Initierung sowie die Analyse der sich potentiell ergebenden abgeleiteten Proteinsequenzen die Identifizierung des authentischen ORF. Dies wird weiter verifiziert durch Generierung von Immunseren gegen abgeleitete Proteine der potentiellen ORFs und ihre Analyse auf Erkennung eines realen Proteins in Geweben und Zelllinien.

### 4. Gewinnung von Antikörpern

Die Charakterisierung der erfindungsgemäß identifizierten Tumor-assoziierten Antigene erfolgt beispielsweise durch die Verwendung von Antikörpern. Ferner umfasst die Erfindung die diagnostische Verwendung von Antikörpern. Dabei können Antikörper Proteine in nativem und/oder denaturierten Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60,1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 989; Gardsvoll, J. Immunol. Methods. 234: 107-116, 2000). Dies ist vor allem für die Herstellung von therapeutischen Antikörpern von Bedeutung, hat aber auch für viele diagnostische Anwendungen eine hohe Bedeutung. Dazu kann sowohl mit dem gesamten Protein als auch mit extrazellulären Teilsequenzen immunisiert werden.

### Immunisierung und Gewinnung von polyklonalen Antikörpern

Verschiedenste Immunisierungsprotokolle sind publiziert. Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der letzten Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonalen Antikörper, mit denen das Zielprotein im Western Blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend). Die Durchführung einer Immunisierung wird als Service von verschiedenen Dienstleistern kommerziell angeboten.
(1) Im ersten Fall werden Peptide (Länge: 8-12 Aminosäuren) über *in vitro* Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung).
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. *E. coli*)*,* in Hefe (z.B. *S. pombe*), in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befinden sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielprotein (Jung et al., Mol. Cells 12: 41-49, 2001; Kasinrerk et al., Hybrid Hybridomics 21: 287-293, 2002).

### Gewinnung monoklonaler Antikörper

Monoklonale Antikörper werden traditionell mit Hilfe der Hybridoma Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM" Technologie eingesetzt. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 5. Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Targetprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene mit transfiziert, um im folgenden Western Blot die Spezifität des analysierten Antikörpers nachweisen zu können.

Im anschließenden Western Blot werden Zellen aus Zellkultur oder Gewebeproben, die das Zielprotein enthalten könnten, in einer 1%eigen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch eines von mehreren Blotting Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im "in silico"-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western Blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristilierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western Blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N- Glykosylasen inkubiert (nach Angaben des jeweiligen Herstellers, z.B. PNgase, Endoglykosidase F, Endoglykosidase H, Roche Diagnostics). Anschließend erfolgt ein Western Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Zielmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of function") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA) Technologie in Zellen erzeugt werden (Hannon, GJ. 2002. RNA interference. Nature 418: 244-51; Czauderna et al. 2003. Nucl. Acid Res. 31: 670-82). Dazu werden Zellen mit kurzen, ca. 20-25 Nukleotide langen, doppelsträngigen RNA Molekülen transfiziert, die für das Zielmolekül spezifisch sind. Ein enzymatischer Prozess führt anschließend zum Abbau der spezifischen RNA des Zielgens, was in einer verminderten Expression des Zielproteins resultiert, und ermöglicht damit die funktionelle Analyse des Zielgens.

### Beispiel 1: Identifizierung von LOC90625 als diagnostisches und therapeutisches Krebs-Target

Das Gen LOC90625 (Nukleinsäuresequenz: SEQ ID NO: 13) ist ein bisher nicht charakterisiertes Gen auf Chromosom 21 (21q22.3). Es kodiert für ein Protein (Aminosäuresequenz: SEQ ID NO:14) mit einer Transmembrandomäne aber ansonsten keinerlei Homologien zu bereits bekannten Proteinen.

Erfindungsgemäß wurde nach der Etablierung einer LOC90625-spezifischen RT-PCR (Primerpaar SEQ ID NO: 15 und 16) die Menge an genspezifischen Transkripten in gesundem Gewebe und in Karzinomproben untersucht (Abb. 1a, b, c). LOC90625 ist in gesundem Gewebe sehr selektiv exprimiert, spezifische Transkripte sind vor allem in dem Testis nachweisbar. Gering oder gar nicht nachweisbar war die LOC90625-spezifische Expression in allen anderen analysierten gesunden Geweben (Abb. 1a, b). Überraschenderweise detektierten wir LOC90625-spezifische Überexpression in verschiedenen Tumortypen. Insbesondere in Prostata-, Ösophagus-, Kopf/Hals- Pankreaskarzinomen und Leukämien war LOC90625 im Vergleich zu den jeweiligen gesunden Gewebeproben stark überexprimiert (Abb. 1a, b, c).

LOC90625 ist ein selektiv exprimiertes Antigen, das offensichtlich in proliferierenden Geweben verstärkt exprimiert wird. Diese selektive Überexpression in Tumoren ist diagnostisch und therapeutisch nutzbar.

Insbesondere die extrazelluläre Domäne von LOC90625 kann erfindungsgemäß als Zielstruktur von monoklonalen Antikörpern genutzt werden. Dies können z.B. 1-19 (SEQ ID NO: 287) oder aber die Aminosäuren 40-160 (SEQ ID NO: 288) sein. Zur Herstellung von LOC203413-spezifischen Antikörpern wurden die Peptide gemäß SEQ ID NO: 289 und 290 verwendet.

Wir konnten nachweisen, dass ein solcher Antikörper spezifisch Zellen, die mit dem genannten Genprodukt transfiziert sind und es exprimieren, anfärben kann (Abb. 2a). Auch weist dieser Antikörper im Western Blot eindeutig ein Protein in der richtigen Größe nach (Abb. 2b). Immunhistochemische Färbungen von Testis als Positivkontrolle aber auch von Prostatatumoren (Abb. 2c) zeigten, dass dieses neu entdeckte Genprodukt tatsächlich zu Protein translatiert wird, dass es nicht in Normalgeweben außer Testis, aber in Tumoren nachweisbar ist, und dass Tumorzellen eines Patienten tatsächlich auf der Oberfläche angefärbt werden. Des weiteren unterstützen diese Daten, dass dieses Genprodukt mit seiner außerordentlichen Tumorzell-Selektivität als Zielstruktur für einen therapeutisch und diagnostisch einsetzbaren Antikörper dienen kann, wie auch dass prinzipiell solche Antikörper gegen dieses Genprodukt generiert werden können.

Therapeutisch sind auch andere zielorientierte Ansätze wie Vakzine und Therapien mit "small compounds" denkbar, die nur dieses Gen als Zielstruktur haben und somit keine gesunden Zellen betreffen. Auch diagnostisch kann dieses Gen aufgrund seiner Selektivität für Tumorzellen genutzt werden.

## Patentansprüche

1. Verfahren zur Diagnose oder Überwachung einer Krebserkrankung, die sich dadurch auszeichnet, dass ein Tumor-assoziiertes Antigen in Tumorgewebe im Vergleich zu vergleichbarem gesunden Gewebe überexprimiert wird, umfassend
(i) den Nachweis einer Nukleinsäure, die für das Tumor-assoziierte Antigen kodiert, und/oder
(ii) den Nachweis des Tumor-assoziierten Antigens,
in einer aus einem Patienten isolierten biologischen Probe,
wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist.

2. Verfahren nach Anspruch 1, wobei der Nachweis der Nukleinsäure mit einer Polynukleotid-Sonde, die spezifisch mit der Nukleinsäure hybridisiert, oder durch selektive Amplifikation der Nukleinsäure erfolgt.

3. Verfahren nach Anspruch 1, wobei der Nachweis des Tumor-assoziierten Antigens mit einem Antikörper erfolgt, der spezifisch an das Tumor-assoziierte Antigen bindet.

4. Verwendung eines Antikörpers, der an ein Tumor-assoziiertes Antigen bindet und mit einem diagnostischen Mittel gekoppelt ist, zur Herstellung einer pharmazeutischen Zusammensetzung zur Diagnose oder Überwachung einer Krebserkrankung, die sich dadurch auszeichnet, dass das Tumor-assoziierte Antigen in Tumorgewebe im Vergleich zu vergleichbarem gesunden Gewebe überexprimiert wird, wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist.

5. Verfahren nach Anspruch 3 oder Verwendung nach Anspruch 4, wobei der Antikörper ein monoklonaler, chimäre oder humanisierter Antikörper oder ein Fragment eines Antikörpers ist.

6. Verfahren oder Verwendung nach einem der Ansprüche 1-5, wobei das Tumor-assoziierte Antigen die Aminosäuresequenz gemäß SEQ ID NO: 14 des Sequenzprotokolls umfasst.

7. Verwendung eines Kits zum Nachweis einer Krebserkrankung, die sich dadurch auszeichnet, dass ein Tumor-assoziiertes Antigen in Tumorgewebe im Vergleich zu vergleichbarem gesunden Gewebe überexprimiert wird, wobei der Kit Mittel zum Nachweis
(i) der Nukleinsäure, die für das Tumor-assoziierte Antigen kodiert, und /oder
(ii) des Tumor-assoziierten Antigens,
umfasst, wobei das Tumor-assoziierte Antigen eine Sequenz aufweist, die von einer Nukleinsäure kodiert wird, die die Nukleinsäuresequenz gemäß SEQ ID NO: 13 des Sequenzprotokolls umfasst, wobei die Krebserkrankung ein Karzinom im Kopf-Hals-Bereich oder eine Leukämie ist.

8. Verwendung nach Anspruch 7, wobei die Mittel zum Nachweis der Nukleinsäure, die für das Tumor-assoziierte Antigen kodiert, Nukleinsäuremoleküle für die selektive Amplifikation der Nukleinsäure sind.

## Claims

1. A method of diagnosing or monitoring a cancer disease **characterized by** an overexpression of a tumor-associated antigen in tumor tissue compared to comparable healthy tissue, which method comprises
(i) detecting a nucleic acid which codes for the tumor-associated antigen, and/or
(ii) detecting the tumor-associated antigen,
in a biological sample isolated from a patient,
said tumor-associated antigen having a sequence encoded by a nucleic acid which comprises the nucleic acid sequence according to SEQ ID NO: 13 of the sequence listing, wherein the cancer disease is a head neck carcinoma or a leukemia.

2. The method of claim 1, wherein the nucleic acid is detected using a polynucleotide probe which hybridizes specifically to said nucleic acid or by selectively amplifying said nucleic acid.

3. The method of claim 1, wherein the tumor-associated antigen is detected using an antibody binding specifically to said tumor-associated antigen.

4. Use of an antibody which binds to a tumor-associated antigen and is coupled to a diagnostic agent for preparing a pharmaceutical composition for diagnosing or monitoring a cancer disease **characterized by** an overexpression of the tumor-associated antigen in tumor tissue compared to comparable healthy tissue, said tumor-associated antigen having a sequence encoded by a nucleic acid which comprises the nucleic acid sequence according to SEQ ID NO: 13 of the sequence listing, wherein the cancer disease is a head neck carcinoma or a leukemia.

5. The method of claim 3 or the use of claim 4, wherein the antibody is a monoclonal, chimeric or humanized antibody, or a fragment of an antibody.

6. The method or the use of any one of claims 1-5, wherein the tumor-associated antigen comprises the amino acid sequence according to SEQ ID NO: 14 of the sequence listing.

7. Use of a kit for detecting a cancer disease **characterized by** an overexpression of a tumor-associated antigen in tumor tissue compared to comparable healthy tissue, which kit comprises agents for detecting
(i) the nucleic acid which codes for the tumor-associated antigen, and/or
(ii) the tumor-associated antigen,
said tumor-associated antigen having a sequence encoded by a nucleic acid which comprises the nucleic acid sequence according to SEQ ID NO: 13 of the sequence listing, wherein the cancer disease is a head neck carcinoma or a leukemia.

8. Use of claim 7, wherein the agents for detecting the nucleic acid which codes for the tumor-associated antigen are nucleic acid molecules for selective amplification of said nucleic acid.

## Revendications

1. Procédé pour le diagnostic ou la surveillance d'une affection cancéreuse **caractérisée par** la surexpression d'un antigène associé à une tumeur dans le tissu tumoral par comparaison avec du tissu sain comparable, comportant
(i) la détection d'un acide nucléique qui code pour l'antigène associé à une tumeur, et/ ou
(ii) la détection de l'antigène associé à une tumeur,
dans un échantillon biologique isolé à partir d'un patient,
l'antigène associé à une tumeur présentant une séquence qui est codée par un acide nucléique qui possède la séquence d'acide nucléique selon SEQ ID NO: 13 du listage de séquences, tandis que l'affection cancéreuse est un carcinome de la tête et du cou ou une leucémie.

2. Procédé selon la revendication 1, dans lequel l'acide nucléique est détecté au moyen d'une sonde polynucléotidique qui hybride spécifiquement audit acide nucléique ou par amplification sélective dudit acide nucléique.

3. Procédé selon la revendication 1, dans lequel l'antigène associé à une tumeur est détecté au moyen d'un anticorps se liant spécifiquement audit antigène associé à une tumeur.

4. Utilisation d'un anticorps qui se lie à un antigène associé à une tumeur et est couplé à un agent de diagnostic pour la préparation d'une composition pharmaceutique pour le diagnostic ou la surveillance d'une affection cancéreuse qui se **caractérise par** une surexpression de l'antigène associé à une tumeur dans un tissu tumoral par comparaison avec un tissu sain comparable, ledit antigène associé à une tumeur ayant une séquence codée par un acide nucléique qui comprend la séquence d'acide nucléique selon SEQ ID NO: 13 du listage de séquences, tandis que l'affection cancéreuse est un carcinome de la tête et du cou ou une leucémie.

5. Procédé selon la revendication 3 ou utilisation selon la revendication 4, dans lequel(laquelle) l'anticorps est un anticorps, ou un fragment d'un anticorps, monoclonal, chimérique ou humanisé.

6. Procédé ou utilisation selon l'une quelconque des revendications 1-5, dans lequel(laquelle) l'antigène associé à une tumeur comprend la séquence d'acides aminés selon SEQ ID NO: 14 du listage de séquences.

7. Utilisation d'un kit pour la détection d'une affection cancéreuse, **caractérisée par** une surexpression d'un antigène associé à une tumeur dans un tissu tumoral par comparaison avec un tissu sain comparable, lequel kit comprend des agents pour la détection de
(i) l'acide nucléique qui code pour l'antigène associé à une tumeur, et/ ou
(ii) l'antigène associé à une tumeur,
ledit antigène associé à une tumeur ayant une séquence codée par un acide nucléique qui comprend la séquence d'acide nucléique selon SEQ ID NO: 13 du listage de séquences, tandis que l'affection cancéreuse est un carcinome de la tête et du cou ou une leucémie.

8. Utilisation selon la revendication 7, dans laquelle les agents pour la détection de l'acide nucléique qui code pour l'antigène associé à une tumeur sont des molécules d'acide nucléique pour l'amplification sélective dudit acide nucléique.
